(19) Europäisches Patentamt
European Patent Office
Office européen des brevets

(11) **EP 2 517 786 A2**

(12) **EUROPÄISCHE PATENTANMELDUNG**

(43) Veröffentlichungstag:
**31.10.2012   Patentblatt 2012/44**

(51) Int Cl.:
**B01J 19/28** (2006.01)   **C12M 1/00** (2006.01)
**B01F 15/00** (2006.01)   **B01F 11/00** (2006.01)
**C12M 3/04** (2006.01)   **G06K 19/07** (2006.01)

(21) Anmeldenummer: **12176531.7**

(22) Anmeldetag: **03.12.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **05.05.2009   DE 102009019697**

(62) Dokumentnummer(n) der früheren Anmeldung(en) nach Art. 76 EPÜ:
**09764729.1 / 2 427 268**

(71) Anmelder: **Bayer Technology Services GmbH**
**51368 Leverkusen (DE)**

(72) Erfinder:
• **Kauling, Joerg**
**51427 Bergisch Gladbach (DE)**

• **Meuser, Bernd**
**50829 Köln (DE)**
• **Braun, Arndt**
**42859 Remscheid (DE)**
• **Kahlert, Wolfgang**
**34327 Körle (DE)**
• **Chaussin, Sebastien**
**13400 Aubagne (FR)**

(74) Vertreter: **Bayer Intellectual Property GmbH**
**Creative Campus Monheim**
**Alfred-Nobel-Straße 10**
**40789 Monheim (DE)**

Bemerkungen:
Diese Anmeldung ist am 16-07-2012 als Teilanmeldung zu der unter INID-Code 62 erwähnten Anmeldung eingereicht worden.

(54) **Reaktor**

(57) Gegenstand der Erfindung ist ein vorzugsweise als Einwegelement ausgeführter Reaktor, ein Container zur Aufnahme des Reaktors, eine Vorrichtung umfassend einen Reaktor und eine Antriebseinheit zur Erzeugung einer rotatorisch-oszillierenden Bewegung des Reaktors, sowie die Verwendung der Vorrichtung zur Kultivierung von Zellen und/oder Mikroorganismen.

Fig. 6

EP 2 517 786 A2

## Beschreibung

Gegenstand der Erfindung ist ein vorzugsweise als Einwegelement ausgeführter Reaktor, ein Container zur Aufnahme des Reaktors, eine Vorrichtung umfassend einen Reaktor und eine Antriebseinheit zur Erzeugung einer rotatorisch-oszillierenden Bewegung des Reaktors, sowie die Verwendung der Vorrichtung zur Kultivierung von Zellen und/oder Mikroorganismen.

Bei der stark regulierten pharmazeutischen Produktion entfällt ein großer zeitlicher, technischer und personeller Aufwand auf die Bereitstellung gereinigter und sterilisierter Bioreaktoren. Um Kreuzkontaminationen bei einem Produktwechsel in einer Multi-Purpose-Anlage oder zwischen zwei Produktchargen sicher zu vermeiden, wird außer der Reinigung eine sehr aufwändige Reinigungsvalidierung benötigt, welche bei einer Prozessadaption ggf: wiederholt werden muss.

Dies gilt sowohl für das Upstream-Processing USP, d.h. die Herstellung biologischer Produkte in Fermentern als auch für das Downstream-Processing DSP, d.h. die Aufreinigung der Fermentationsprodukte.

Im USP und DSP kommen dabei häufig Kessel als Rühr- und Reaktionssysteme zum Einsatz. Gerade bei der Fermentation ist eine keimfreie Umgebung für eine erfolgreiche Kultivierung essentiell. Zur Sterilisation von Batch- oder Fed-Batch-Fermentern kommt in der Regel die SIP-Technik zum Einsatz (SIP = sterilizable-in-place). Um bei kontinuierlicher Prozessführung eine ausreichende Langzeitsterilität zu gewährleisten wird auch die Autoklavier-Technik genutzt, die allerdings einen umständlichen Transport der Reaktoren zum Autoklaven erfordert und nur in vergleichsweise kleinen Reaktormaßstäben anwendbar ist. Die Gefahr der Kontamination während der Fermentation ist besonders kritisch bei der Probenahme und an bewegten Rührerwellen. Letztere sind in der Regel mit aufwändigen Dichtungssystemen (z.B.: Gleitringdichtungen) ausgestattet. Technologien, die ohne solche Durchdringungen der Fermentationshülle auskommen, werden wegen ihrer größeren Prozessrobustheit bevorzugt.

Der durch die Bereitstellungsprozeduren bedingte Nutzungsausfall der Reaktoren kann insbesondere bei kurzen Nutzungsperioden und häufigem Produktwechsel in der Größenordnung der Reaktorverfügbarkeit liegen. Betroffen sind im USP der biotechnologischen Produktion z.B. die Prozessschritte der Medienherstellung und Fermentation und im DSP das Solubilisieren, Einfrieren, Auftauen, pH-Adjustieren, Fällen, Kristallisieren, das Umpuffern und die Virusinaktivierung.

Um der Forderung an ein schnelles und flexibles Neubeschicken der Produktionsanlage unter Wahrung maximaler Sauberkeit und Sterilität gerecht zu werden, erfreuen sich auf dem Markt Konzepte für Einwegreaktoren eines ständig wachsenden Interesses.

In WO2007/121958A1 ist ein solcher Einwegreaktor für die Kultivierung von Zellen und Mikroorganismen beschrieben. Er besteht in einer bevorzugten Ausführungsform aus einem stabilen, vorzugsweise mehrlagigen Polymerwerkstoff. Der verformbare Einwegreaktor wird von einem Behälter aufgenommen, der ihn stützt. Dabei wird er bevorzugt von oben in den Behälter eingeführt. Der Behälter ist mit einer Antriebseinheit verbunden. Durch die Antriebseinheit wird der Behälter inklusive des Einwegreaktors in eine rotatorisch-oszillierende Bewegung um eine ortsfeste, vorzugsweise vertikale Achse des Behälters versetzt. Durch eine eckige Ausführungsform des Einwegreaktors und/oder Einbauten im Einwegreaktor kann bei der oszillatorisch rotierenden Bewegung ein hoher Leistungseintrag in den Reaktorinhalt erreicht werden, so dass der Einwegreaktor als oberflächenbegaster Fermenter zur Kultivierung von Zellen und Mikroorganismen eingesetzt werden kann.

Der in WO2007/121958A1 beschriebene Einwegreaktor weist einen flachen Boden oder einen nach außen gewölbten pyramidalen Boden mit zentralem Ablauf auf. Durch den nach außen gewölbten pyramidalen Boden mit zentralem Ablauf soll erreicht werden, dass nach Öffnung eines Ventils im Ablauf eine vollständige Entnahme des Reaktorinhalts über eine Schlauchleitung möglich ist.

Nachteilig an dem in WO2007/121958A1 beschriebenen System ist, dass bei zunehmender Größe des Einwegreaktors Probleme auftreten, die bei einem Reaktorvolumen von 10 L bis 100 L eine untergeordnete Rolle spielen.

Ein flacher Boden hat beispielsweise den Nachteil, dass der Einwegreaktor nicht ohne Weiteres vollständig entleert werden kann: bei einem Ablauf oberhalb der Bodenkante verbleibt ein Teil des Reaktorinhalts im Reaktor. Bei einem großvolumigen Reaktor können dies beträchtliche Mengen an Produkt sein. Der nach außen gewölbte Boden mit zentralem Ablauf bietet hier den Vorteil, dass der Reaktor nahezu vollständig entleert werden kann.

Allerdings wird es mit zunehmender Größe des Einwegreaktors zunehmend schwieriger, diesen von oben in einen Behälter einzuführen. Weiterhin wird es zunehmend schwieriger, eine Schlauchleitung von außen an den zentralen Ablauf des pyramidal nach außen gewölbten Bodens des Einwegreaktors anzubringen. Darüber hinaus stellt eine nach unten geführte Schlauchleitung ein Sicherheitsrisiko dar: bei einer undichten Verbindung oder einem Platzen der Schlauchleitung läuft der gesamte Reaktorinhalt ungehindert aus.

Mit zunehmender Größe des Reaktors sinkt zudem die Mischwirkung der rotatorisch-oszillierenden Bewegung in der Nähe der Rotationsachse. Es besteht die Gefahr, dass bei der Kultivierung von Zellen und/oder Mikroorganismen die in der Nähe der Rotationsachse befindlichen Kulturen keine ausreichende Nährstoffversorgung erhalten.

Durch das mit zunehmender Reaktorgröße abnehmende Oberflächen/Volumen-Verhältnis besteht insbesondere bei größeren Reaktoren die Gefahr einer unzureichenden Versorgung von Zellen und/oder Mikroorganismen mit Gasen, wenn diese ausschließlich durch Oberflächenbegasung in den Reaktor eingebracht werden.

Es stellt sich ausgehend vom Stand der Technik die Aufgabe, ein System zur Durchführung von Prozessen mit hohen Anforderungen an Reinheit und Sterilität bereitzustellen, das den zeitlichen, technischen und personellen Aufwand auf die Bereitstellung gereinigter und sterilisierter Komponenten reduziert. Das System soll insbesondere auch für Prozessvolumina von 100 L bis 200 L und größer verwendbar sein. Es soll den hohen Anforderungen der pharmazeutischen Industrie gerecht werden, einfach und intuitiv zu handhaben und kostengünstig sein. Es soll Sicherheitsrisiken durch das Austreten von Stoffen aus dem Prozessraum auf ein Minimum reduzieren. Es soll eine ausreichende Durchmischung des Prozessinhalts ermöglichen. Es soll für die Kultivierung von Mikroorganismen und Zellkulturen geeignet sein und hierbei für eine ausreichende Ver- und Entsorgung des Kulturmediums mit insbesondere gasförmigen Stoffen sorgen.

Erfindungsgemäß wird diese Aufgabe durch die in den unabhängigen Ansprüchen aufgeführten Gegenstände gelöst. Bevorzugte Ausführungsformen finden sich in den abhängigen Ansprüchen.

Ein erster Gegenstand der vorliegenden Erfindung ist ein Reaktor umfassend Wände, die einen Innenraum einschließen, und Durchführungen und/oder Anschlüsse in den Wänden, die eine Verbindung des Innenraums mit der äußeren Umgebung gestatten, dadurch gekennzeichnet, dass der Reaktor in mit Flüssigkeit gefülltem Zustand einen parallel zur Flüssigkeitsoberfläche eckigen Querschnitt und einen in den Innenraum gewölbten oder wölbbaren Boden aufweist.

Der erfindungsgemäße Reaktor stellt einen nach außen abdichtbaren Raum für die Durchführung von chemischen, biologischen, biochemischen und/oder physikalischen Prozessen dar. Insbesondere dient der Reaktor der Bereitstellung eines Raumes zur Kultivierung von Zellen und/oder Mikroorganismen.

Der Reaktor ist vorzugsweise als Einwegelement ausgeführt, d.h. es ist vorzugsweise vorgesehen, den Reaktor nach der Verwendung nicht zu reinigen sondern zu entsorgen. Daher umfasst der Reaktor nur die wesentlichen Elemente, die zur Bereitstellung eines sterilen Reaktionsraumes notwendig sind: Wände, die einen Raum einschließen und diesen nach außen abdichten, und Durchführungen in den Wänden, um den Reaktionsraum mit der Außenwelt in kontrollierter Weise verbinden zu können. Alle übrigen Elemente, die zum Betrieb eines Reaktors, insbesondere zur Kultivierung von Zellen und/oder Mikroorganismen benötigt werden, werden durch eine Peripherie bereitgestellt, die wiederverwendbar ist. Diese Peripherie, die einen Container zur Aufnahme des Reaktors und eine Antriebseinheit zur rotatorisch-oszillierenden Bewegung des Reaktors umfasst, ist weiter unten näher beschrieben.

Der Reaktor, der im Stand der Technik üblicherweise eine zusammenhängende Einheit darstellt, wird also im vorliegenden Fall vorzugsweise in separate Teile aufgeteilt, die ihren Funktionen gemäß gestaltet sind. Der Reaktor und der Container sind als separate Teile eines Gesamtsystems dabei so aufeinander angepasst, dass der Reaktor in den Container eingebracht werden kann und von diesem im flüssigkeitsgefüllten Zustand gestützt wird. Der Reaktor kann als innere Haut des Containers aufgefasst werden, die erneuerbar ist, um für einen Prozesswechsel eine ungebrauchte, sterile Reaktionszelle zu gewährleisten.

Der erfindungsgemäße Reaktor ist so ausgeführt, dass er kostengünstig hergestellt werden kann. Vorzugsweise ist der Reaktor ein verformbarer, hohler Körper, dessen Wände flexibel, d.h. biegsam sind, und dessen Form sich zumindest im ungefüllten Zustand des Reaktors durch eine äußere Kraft verändern lässt. Die Wände bestehen vorzugsweise aus einem stabilen, ein- oder mehrlagigen Polymerwerkstoff. Durch die Verformbarkeit ist es beispielsweise möglich, den Reaktor zu falten und somit in eine platzsparende Form für Transport und Lagerung zu bringen.

Die Verformbarkeit ergibt sich bei einem vorzugsweise als Einwegelement ausgeführten Reaktor auch aus der Forderung nach geringen Material- und Herstellungskosten für den Reaktor. Da der Reaktor im wesentlichen nur einen sterilen Raum bereitstellen soll, müssen die Wände beispielsweise nicht selbsttragend sein. Stattdessen wird der Reaktor als verformbarer Körper ausgeführt, der zur Verwendung in einen Container eingebracht wird, der den Reaktor stützt. Somit können die Wandstärken des Reaktors auf ein Minimum reduziert wird, das den Stabilitätskriterien für einen gestützten Reaktor gerecht wird. Üblichweise liegen die Wandstärken damit in einem Bereich, in dem die Wände verformbar sind.

Überdies kann sich ein verformbarer Reaktor mit flexiblen Wänden an die Bereiche eines Containers, die den Reaktor bei dessen Verwendung stützen sollen, anschmiegen, was z.B. vorteilhaft für das Ausgleichen von Fertigungstoleranzen sein kann.

Die Wände des erfindungsgemäßen Reaktors (und damit vorzugsweise auch die zur Aufnahme des Reaktors dienenden Bereiche des Containers) sind so angeordnet, dass bei einer rotatorisch-oszillierenden Bewegung des Reaktors um eine ortsfeste Achse ein Leistungseintrag in den flüssigen Inhalt des Reaktors erfolgt.

Wäre der Reaktor als ein Kreiszylinder ausgeführt und würde der mit Flüssigkeit gefüllte Reaktor eine rotatorisch-oszillierende Bewegung um die Zylinderlängsachse vollführen, so würde sich die träge Flüssigkeit im Inneren des Reaktors kaum mit dem Reaktor mitbewegen. Lediglich die wandnahen Schicht würden durch die Adhäsionsskräfte zu einer Mitrotation gezwungen werden. Daher weist der erfindungsgemäße Reaktor im flüssigkeitsgefüllten Zustand einen eckigen Querschnitt parallel zur Flüssigkeitsoberfläche auf. Die eckige Ausführungsform sorgt für einen Leistungseintrag in die Flüssigkeit und prägt dem Reaktorinhalt die Bewegung des Reaktors auf. Die Bewegung des Reaktors erfolgt oszillierend, d.h. die Drehrichtung wird periodisch gewechselt. Aufgrund der Trägheit und des fluiden Zustands des Reaktorinhalts hinkt die Bewegung des Reaktorinhalts der Bewegung des Reaktors nach. Hierdurch wird eine Mischwirkung erzielt. Insbesondere ist es hierdurch möglich, Gas oberhalb der Flüssigkeit in die Flüssigkeit einzutragen. Dies ist beispielhaft auf Seite 25 und in Figur 5c der Offenlegung WO2007/121958A1 dargelegt.

Der Querschnitt parallel zur Flüssigkeitsoberfläche des gefüllten Reaktors weist vorzugsweise die Form eines n-Ecks mit n im Bereich von 3 bis 12 auf, bevorzugt im Bereich von 4 bis 8, ganz besonders bevorzugt im Bereich 4 bis 6, am meisten bevorzugt ist n gleich 4.

Vorzugsweise sind die Seitenwände des erfindungsgemäßen Reaktors zumindest teilweise als ebene Flächen ausgebildet, die nicht parallel zur Flüssigkeitsoberfläche verlaufen und in einem Winkel von 45° bis 120° aufeinandertreffen.

Es ist zum Beispiel denkbar, dass die Seitenwände des Reaktors einen Polyeder bilden.

Der erfindungsgemäße Reaktor weist ferner einen in den Innenraum des Reaktors gestülpten oder im Falle eines verformbaren Reaktors einen nach innen stülpbaren Boden auf. Der Boden kann zum Beispiel die Form eines nach innen gerichteten Tetraeders, einer nach innen gerichteten Pyramide, die Form eines Paraboloids oder eine Glokkenform aufweisen. Weitere Formen sind denkbar.

Der Boden weist vorzugsweise nach innen gerichtete Kanten auf, die bei einer rotatorisch-oszillierenden Bewegung einen Leistungseintrag in das im Reaktor befindliche Fluid bewirken. Besonders bevorzugt ist der Boden pyramidal ausgeführt.

Der in den Innenraum gestülpte oder stülpbare Bereich wird im Folgenden auch als Wölbung bezeichnet.

Die Höhe $h$ der Wölbung liegt im Bereich des 0,01-fachen bis 1-fachen des kreisäquivalenten Durchmessers $d$ des Bodenquerschnitts. Ist der Bodenquerschnitt beispielsweise quadratisch mit einer Seitenlänge $a$, wie dies bei einem pyramidal gewölbten Boden der Fall sein kann, so beträgt die Querschnittsfläche $A=a^2$. Der kreisäquivalente Durchmesser d beträgt dann nach Formel 1:

$$d = \sqrt{\frac{4A}{\pi}} \qquad (1)$$

Bevorzugt liegt die Höhe $h$ der Wölbung zum kreisäquivalenten Durchmesser d im Bereich von 3% bis 100%, besonders bevorzugt im Bereich von 5% bis 30% und ganz besonders bevorzugt im Bereich von 10% bis 20%.

Überraschend wurde gefunden, dass ein nach innen gewölbter/gestülpter Boden, insbesondere wenn er polyederförmig (z.B. pyramidal) ausgeführt ist und damit über in das Reaktorinnere gerichtete Kanten verfügt, die Mischwirkung des rotatorisch-oszillierenden Reaktors gegenüber einem flach ausgeführten oder nach außen gestülpten Boden erheblich verbessert. Dies ist auf einen durch die nach innen gerichteten Kanten erhöhten Leistungseintrag bei der rotatorisch-oszillierenden Bewegung zurückzuführen. Weiterhin bewirkt eine Bodenform mit nach innen gerichteten Kanten eine Re-Suspendierung von Partikeln, die im Reaktorinnern zu Boden sinken. Durch die oszillatorische Bewegung entstehen an den Kanten Wirbel, die Partikel wieder zurück in den oberen Bereich des Reaktors fördern.

Ferner reduziert die Innenwölbung das Restvolumen bei einer Entnahme des Reaktorinhalts über seitliche Anschlüsse oberhalb der Bodenkante des Reaktors. Auf diese Weise lassen sich in den Boden hineinreichende Drainage-Anschlüsse und die hiermit verbundenen Probleme und Risiken vermeiden.

Der erfindungsgemäße Reaktor verfügt über Durchführungen und/oder Anschlüsse, um beispielsweise Stoffe ab- und/oder zuführen und/oder Messungen am Reaktorinhalt durchführen zu können.

Da der Reaktor eine rotatorisch-oszillierende Bewegung um eine ortsfeste Achse vollführen soll, ist man geneigt, Durchführungen und/oder Anschlüsse in die Nähe der Rotationsachse zu bringen, um beispielsweise Schlauchlängen für die Zu- und/oder Abfuhr von Stoffen möglichst kurz zu halten. Schläuche können in dem Fall einfach entlang der Rotationsachse vom Reaktor weggeführt werden und verheddern sich nicht so schnell. Auch in der Offenlegung WO2007/121958A1 werden Schläuche vorzugsweise im Kopfbereich des Reaktors nahe der Rotationsachse angebracht.

Es hat sich jedoch gezeigt, dass Anschlüsse im Kopfbereich nachteilig sind, insbesondere wenn es sich um Schlauchenden handelt, die von oben in den Reaktorinhalt hineinragen sollen (dip tubes), da diese aufgrund der rotatorisch-oszillierenden Bewegung keine reproduzierbare Position im Reaktor einnehmen, sondern vielmehr hin- und herschlenkern. Solche Schlauchenden müssen schon bei der Herstellung des Reaktors in das Reaktorinnere eingebracht werden, was zu erhöhten Herstellkosten führt. Die Schlauchenden können bei der Herstellung des Reaktors, beim Transport, bei der Inbetriebnahme und während des Betriebs leicht verheddern. Sie sind von außen nicht oder nur sehr schwer entwirrbar, da das Reaktorinnere, insbesondere während des Betriebs, von außen nicht zugänglich ist.

Daher weist der erfindungsgemäße Reaktor vorzugsweise Durchführungen und/oder Anschlüsse im Seitenbereich auf.

Eine Durchführung zum Ablassen des Reaktorinhalts ist vorzugsweise nahe der Bodenkante angebracht.

Über die ebenfalls seitlich angebrachten Anschlüsse kann beispielsweise der pH-Wert und/oder die Sauerstoffkonzentration im Reaktor gemessen werden. Besonders geeignet sind Messsysteme, die berührungslos arbeiten, wie Fluoreszenzsensoren, die optisch abgefragt werden können (siehe hierzu auch WO2007/121958A1, in der solche Sensoren näher beschrieben sind).

Der erfindungsgemäße Reaktor ist vorzugsweise aus einem ein- oder mehrlagigen durchsichtigen Polymermaterial ausgeführt, dass Einblick in den Reaktor während des Betriebs ermöglicht.

Polymermaterial ist ein vergleichweise kostengünstiges Material, das sich auch vergleichsweise kostengünstig verarbeiten lässt. Die Entsorgung des gebrauchten Reaktors und die Verwendung eines neuen Einwegreaktors ist damit wirtschaftlicher als die Reinigung von gebrauchten Reaktoren, insbesondere da bei der Verwendung eines neuen Einwegreaktors eine aufwändige Reinigungsvalidierung entfällt. Der erfindungsgemäße Reaktor ist vorzugsweise steril verpackt.

Als Materialien für den erfindungsgemäßen Reaktor eignen sich insbesondere die in der Patentschrift US6,186,932B1 in den Spalten 2 und 3 für die dort genannten Transportbeutel (*sachets*) verwendeten Materialien und Materialkombinationen. Auch die dort aufgeführten Wandstärken lassen sich auf erfindungsgemäße Reaktoren übertragen.

In einer bevorzugten Ausführungsform bestehen die Wände des erfmdungsgemäßen Reaktors aus einem Folienverbund umfassend eine Schicht aus Polypropylen und eine Schicht aus Polyethylen.

Die Polyethylenschicht befindet sich vorzugsweise auf der Reaktorinnenseite, während sich die Polypropylenschicht vorzugsweise auf der Reaktoraußenseite befindet.

Der erfindungsgemäße Reaktor aus Polymerfolien kann beispielsweise nach dem in US6,186,932B1 beschriebenen Verfahren hergestellt werden, wobei die Schweißnähte so angepasst werden müssen, dass ein nach innen stülpbarer Boden entsteht. Ein Ausführungsbeispiel zur Herstellung einer bevorzugten Ausführungsform eines erfindungsgemäßen Reaktors ist weiter unten beschrieben.

Der erfindungsgemäße Reaktor weist ein Verhältnis von Höhe zur maximalen Breite im Bereich von 0,2 bis 3, bevorzugt 0,5 bis 2, besonders bevorzugt 0,7 bis 1,5 auf.

Das Reaktorvolumen kann beispielsweise Werte von 10 L bis 300 L annehmen.

Zur bestimmungsgemäßen Verwendung wird der Reaktor vorzugsweise in einen Container eingebracht, der die flexiblen Wände des Reaktors stützt, wenn der Reaktor gefüllt ist. Daher sind die Formen von Container und Reaktor vorzugsweise aufeinander abgestimmt.

Der Container zur Aufnahme eines erfindungsgemäßen Reaktors ist ein weiterer Gegenstand der vorliegenden Erfindung.

Der erfindungsgemäße Container umfasst mindestens

- einen Innenraum zur Aufnahme eines Reaktors,

- eine Öffnung zur frontalen Einführung eines Reaktors in den Container, wobei die Öffnung schließbar und im geschlossenen Zustand flüssigkeitsdicht ausgeführt ist,

- einen nach innen in den Innenraum des Containers gewölbten Boden, und

- einen Seitenkanal, über den Schläuche und/oder Kanäle und/oder Messsonden an den Reaktor herangeführt werden können.

Unter Container wird ein Behälter verstanden, der einen Innenraum einschließt und im geschlossenen Zustand den Innenraum durch Wände von der Außenwelt abgrenzt. Der erfindungsgemäße Container ist bevorzugt flüssigkeitsdicht ausgeführt, d.h. er kann gegenüber der Außenwelt so abgedichtet werden, dass keine Flüssigkeit ungewollt aus dem Inneren des Containers in die Außenwelt gelangt.

Der Container kann zylinderförmig, z.B. in Form einer Säule, oder als Quader ausgeführt sein. Der Container ist bevorzugt quaderförmig ausgeführt. Breite und Tiefe des Containers sind bezogen auf den Innenraum ohne Seitenkanal besonders bevorzugt etwa gleich groß, d.h. sie weichen maximal um 200% voneinander ab. Das Verhältnis von Höhe zur maximalen Breite beträgt 0,2 bis 3, bevorzugt 0,5 bis 2 und besonders bevorzugt 0,7 bis 1,5. Die zum Teil vergleichsweise kleinen Verhältnisse von Höhe zu Breite erlauben die Unterbringung großer Volumina in niedrigen Räumen, z.B. in Laboratorien, und verbessern den Stofftransport bei Oberflächenbegasung infolge der vergleichsweise hohen spezifischen Oberfläche der Flüssigkeit pro Reaktorvolumen.

In den Innenraum des Containers kann ein erfindungsgemäßer Reaktor eingebracht werden. Der vorzugsweise verformbare Reaktor stützt sich an den Innenwänden des Containers ab. Der Container verfügt über einen nach innen gewölbten Boden. Die Bodenformen des Containers und des Reaktors sind aufeinander abgestimmt.

Die Innenwölbung hat neben der verbesserten Mischwirkung und dem geringeren Totvolumen beim Ablassen des Reaktorinhalt zusätzlich den Vorteil, dass sie die Stabilität des Containers erhöht. Dadurch können die Containerwandstärken reduziert und eine Gewichts- und damit Kosteneinsparung erreicht werden.

Die Deckelplatte des Containers kann je nach Anforderungen an den Druck im Kopfraum so ausführt sein, dass Kräfte vom Reaktor auf den Container übertragen werden können. In diesem Fall ist für einen weitestgehenden Kraftschluss zwischen Reaktor und Container Sorge zu tragen.

Der Container verfügt über mindestens eine an einer Seite des Containers angebrachte Öffnung, über die der Reaktor vorzugsweise frontal in den Container eingebracht werden kann. Im Unterschied zu dem in WO2007/121958A1 beschriebenen System wird der Reaktor vorzugsweise nicht von oben sondern frontal in den Container einbracht. Bei Reaktoren mit Volumina von 100 L bis 200 L und größer erreicht der Container eine solche Höhe, dass ein Einbringen des Reaktors von oben zum einen eine entsprechende Raumhöhe und zum anderen Hilfsmittel zum Erreichen einer oberen Containeröffnung (Leiter, Kran) erfordert. Eine frontal gerichtete Öffnung ist aufgrund der einfacheren Handhabung vorteilhaft.

Die Öffnung ist schließbar ausgeführt, d.h. es ist eine Tür oder dergleichen vorhanden, um den Container vorzugsweise flüssigkeitsdicht schließen zu können.

Der Container verfügt weiterhin über einen Seitenkanal, über den Schläuche und andere Verbindungen wie beispielsweise Sensoren bzw. Sensorleitungen an den Reaktor herangeführt werden können. Der Kanal ist nach außen abgedichtet, so dass im Falle einer Leckage keine Medien, insbesondere keine biologischen Medien mit gentechnisch veränderten Organismen, aus dem Container entweichen können.

In einer bevorzugten Ausführungsform wird der Kanal durch zwei beabstandete, vorzugsweise transparente Wände gebildet, die bis in den Bodenbereich des Einwegreaktors verlaufen. Die innere zum Reaktor gerichtete Wand dient der Stützung des Reaktors. Die äußere Wand dient der Abdichtung des Containers nach außen. Beide Wände lassen sich zur frontalen Einführung des Reaktors in den Container entfernen. Die Schlauchleitungen und anderen Verbindungen werden durch den Kanal nach oben und oberhalb des Reaktors zur Drehachse des Containers geführt und über die Vertikalachse aus der Anlage herausgeführt. Ein vollständiges Auslaufen des Reaktors infolge einer Leckage in einer Verbindung wird somit sicher vermieden. Eine zusätzliche Absicherung der Leitungen gegen die Umgebung ist somit nicht erforderlich.

Der Container verfügt vorzugsweise über Mittel zur Temperierung des Containers und des in dem Container befindlichen Reaktors. Vorzugsweise sind Heiz- und/oder Kühlelemente in oder an Wände des Containers ein-/angebracht, die eine Temperierung ermöglichen.

Der Reaktor oder der Container sind im Betrieb drehbar um eine vertikale Achse gelagert und mit einer Antriebseinheit verbunden. Durch die Antriebseinheit kann der Reaktor um die ortsfeste, vertikale Achse rotatorisch-oszillierend in Bewegung versetzt werden, so dass eine direkte Koppelung der Antriebseinheit mit dem Reaktor selbst nicht erforderlich ist. Durch die Zwangskopplung kann auch die Freisetzung von elektromagnetischen Strahlen, die z.B. Störungen von Sensoren verursachen können, drastisch reduziert werden.

Vorzugsweise wird für die Realisierung der oszillatorischen Reaktorbewegung ein Pendelgetriebe verwendet, das über ein geeignetes Kopplungsmittel (z.B. über einen Zahnriemen) mit dem Reaktor oder dem Container verbunden ist. Vorzugsweise erfolgt die Kopplung von Pendelgetriebe und Reaktor/Container unterhalb des Reaktor-/Containerbodens. Eine solche Anordnung besitzt die Vorteile sehr geräuscharm zu sein und einen niedrigen Schwerpunkt zu ermöglichen. Letzteres ist insbesondere bei großen Apparatevolumina und begrenzter Gebäudehöhe (z.B. bei Aufstellung in Laboratorien) relevant.

Der Container kann beispielsweise von oben mit Hilfe eines Krans in eine Halterung oder ein Axiallager eingesetzt werden, so dass für verschiedene Container-Typen dieselbe Antriebseinheit und/oder dieselbe Messtechnik verwendet werden kann.

Vorzugsweise wird der Reaktor derart mit der Antriebseinheit zwangsgekoppelt, dass das Beschleunigen und Abbremsen der Reaktorrotation mit einer im Wesentlichen konstanten Winkelbeschleunigung bzw. -verzögerung erfolgt. Dadurch werden in jeder Phase der rotatorisch-oszllierenden Reaktorbewegung momentane Spitzenwerte der hydrodynamischen Scherkräfte auf suspendierte Partikel (z.B. tierische Zellen) vergleichsweise geringer gehalten als bei anderen Bewegungsformen des Reaktors. Weiche Übergänge zwischen Beschleunigen und Abbremsen bis zum Extremfall eines sinusförmigen Beschleunigungs- und Verzögerungsverlaufes sind dabei durchaus erwünscht, um die Betriebsdauer der Antriebselemente zu erhöhen. Durch die permanente Wirkung einer Beschleunigung oder Verzögerung ändert sich die Drehgeschwindigkeit des Reaktors in jeder Bewegungsphase der rotatorischen Oszillation mit der Zeit. Zwischengeschaltete Steuermodule sind bei dieser einfachen Reaktorbewegung nicht erforderlich.

Der erfindungsgemäße Container verfügt wie bereits ausgeführt über einen nach innen in den Innenraum des Containers gewölbten und an den erfindungsgemäßen Reaktor angepassten Boden. Der Boden kann dementsprechend wie beim Reaktor kegelförmig, kugelsegmentartig, glockenförmig, parabolisch oder polyederförmig ausgeführt sein.

Bevorzugt weist der Boden nach innen in das Containerinnere gerichtete Kanten auf. Besonders bevorzugt ist der Boden pyramidal ausgeführt.

Die Höhe *h* der Wölbung des Containerbodens liegt im Bereich des 0,01-fachen bis 1-fachen des kreisäquivalenten Durchmessers *d* des Bodenquerschnitts. Ist der Bodenquerschnitt beispielsweise quadratisch mit einer Seitenlänge *a*, wie dies bei einem pyramidal gewölbten Boden der Fall sein kann, so beträgt die Querschnittsfläche $A = a^2$. Der kreisäquivalente Durchmesser *d* beträgt dann nach Formel 1:

$$d = \sqrt{\frac{4A}{\pi}} \qquad (1)$$

Bevorzugt liegt die Höhe *h* der Wölbung zum kreisäquivalenten Durchmesser *d* im Bereich von 3% bis 100%, besonders bevorzugt im Bereich von 5% bis 30% und ganz besonders bevorzugt im Bereich von 10% bis 20%.

Durch den nach innen gewölbten Boden und die bevorzugt quaderförmige Ausführungsform des Containers können in Kombination mit der rotatorisch-oszillierenden Bewegung Verteilungsprozesse und/oder Mischreaktionen auf einfache Weise und mit gleicher Intensität wie in einem konventionellen Rührbehälter durchgeführt werden. Auf eine Wellendurchführung kann bei dem erfindungsgemäßen Konzept hingegen völlig verzichtet werden.

Die Innenwölbung verhindert damit einerseits die Sedimentation von Partikeln im bewegten Zustand durch einen zusätzlichen Leistungseintrag im Bodenbereich. Andererseits reduziert sie das Restvolumen bei einer Entnahme des Reaktorinhalts über seitliche Anschlüsse oberhalb der Bodenkante des Reaktors. Auf diese Weise lassen sich in den Boden hineinreichende Drainage-Anschlüsse vermeiden.

Durch das Konzept des nach innen gewölbten Containerbodens lässt sich, wie bereits oben angesprochen, zudem die Stabilität des Containers erheblich vergrößern. Dadurch können die Wandstärken der Mantelflächen reduziert und eine Gewichts- und damit Kosteneinsparung erreicht werden. Ferner werden durch die Reduzierung der Wandstärken deren Trägheitskräfte und letztlich die Antriebsleistung für den Container erheblich reduziert.

In einer besonders bevorzugten Ausführungsform befindet sich vorzugsweise im Bodenbereich der inneren Wand des Seitenkanals eine Anschlussplatte, die im Bereich der Anschlussleitungen eine Halterung des Reaktors und ein Hindurchführen der Anschlussleitungen in den Seitenkanal ermöglicht.

In einer bevorzugten Ausführungsform besitzt diese Anschlussplatte Durchführungen für das Hindurchtreten der Anschlussleitungen. Sie wird bei der Montage mit dem Reaktor verbunden. In dieser Ausführungsform ist die Anschlussplatte vorzugsweise ebenfalls ein Einwegelement.

In einer weiteren bevorzugten Ausführungsform ist die Anschlussplatte seitlich geöffnet und wird zur Fixierung des verformbaren Reaktors im Container seitlich über die Anschlüsse des Reaktors geschoben. In diesem Fall kann die Anschlussplatte wiederverwendet werden und ist Teil des Containers.

Ein weiterer Gegenstand der vorliegenden Erfindung ist eine Vorrichtung mindestens umfassend einen erfindungsgemäßen Reaktor und eine Antriebseinheit. Ist der Reaktor verformbar ausgeführt und benötigt daher einen Container zur Stützung der Reaktorwände, so umfasst die erfindungsgemäße Vorrichtung weiterhin einen erfindungsgemäßen Container.

Die Vorrichtung weist in einer bevorzugten Ausführungsform ein Gestell auf, das den im Betrieb oszillatorisch rotierenden Reaktor (Container) umgibt. In einer bevorzugten Ausführungsform weist das Gestell eine Tür auf, die bei Betrieb des Reaktors unter Verwendung eines kodierten, fälschungssicheren Schlüsselsystems verriegelt ist. Dieses wird bevorzugt unter Wahrung maximaler Sicherheitsanforderungen an den Arbeitsschutz ohne Stromzufuhr und damit ohne kostenaufwändige elektronische Sensorüberwachung in Gestell und Steuerung eingesetzt. Die Gestellmontage wird dadurch erheblich vereinfacht. Das Gestell weist eine Tür auf, die dem Bedienungspersonal nur bei gesichertem Betriebsstillstand den Zutritt zum Reaktor gewährt. Die Tür und Antriebseinheit sind durch das Schließsystem derart abgesichert, dass eine Inbetriebnahme des Reaktors nur bei geschlossener Tür möglich ist. Bevorzugt wird dazu ein stromloses Sicherungssystem, dem Fachmann auch als Fortis-System bekannt, verwendet. Ein einziger verfügbarer, dedizierter Anlagenschlüssel dient dem Verschließen der Tür und der Steuerung der Antriebseinheit. Nur bei geschlossener Tür kann die Antriebseinheit gestartet werden. Beim Entfernen des Schlüssels aus der Steuereinheit der Antriebseinheit wird die Antriebseinheit abgeschaltet. Der Vorteil dieser kodierten Einschlüsselverriegelung liegt in einer mechanischen Eigensicherheit ohne elektronische Überwachung. Eine Kabelverlegung durch die Hohlrohre des Gestells ist nicht notwendig. Dadurch ergeben sich Vorteile bei Reinigung, Abdichtung, Montage und Kosten.

Gegenstand der vorliegenden Erfindung ist auch die Verwendung einer Vorrichtung umfassend einen Reaktor, ggf. einen Container und eine Antriebseinheit zur Kultivierung von Zellen und/oder Mikroorganismen.

In einer bevorzugten Ausführungsform wird ein verformbarer erfindungsgemäßer Reaktor zunächst in einen auf den Reaktor abgestimmten erfindungsgemäßen Container eingebracht. Vorzugsweise wird der verformbare Reaktor mittels eines Gases expandiert und im halb gefüllten Zustand durch die Öffnung des Containers in den Container eingeführt. Nachdem der Reaktor positioniert und durch die Öffnung arretiert wurde, erfolgt die Füllung mit Medium unter Verdrängung des Gases.

Vorzugsweise wird der Reaktor über die beschriebene Anschlussplatte mit Schlauchleitungen für z.B. Nährmittelversorgung, ggf. eine Gaszufuhr für die Blasenbegasung, die Drainage und weiteren Verbindungen z.B. für Temperatursensor, pH-Sensor und dergleichen verbunden. Die Gasabfuhr aus dem Kopfraum sowie die Gaszufuhr für die Oberflächenbegasung erfolgen bevorzugt am Kopf des Reaktors.

Im Einwegreaktor liegt ein Verhältnis von Flüssigkeitsspiegel zur Reaktorbreite von bevorzugt 0,2 bis 2 und besonders bevorzugt 0,5 bis 1 vor. Außerdem wird der Reaktor unter Wahrung seiner bevorzugten hydrodynamischen und prozesstechnischen Eigenschaften mit ausreichendem Kopfraum zwischen Reaktorkopf und Flüssigkeitsspiegel von mindestens 5% bis 50 % Flüssigkeitshöhe bevorzugt mindestens 25% Flüssigkeitshöhe betrieben.

Die mit dem Reaktor oder Container verbundene Antriebseinheit sorgt durch eine rotatorisch-oszillierende Bewegung für eine gute Durchmischung des Reaktorinhalts.

Es hat sich überraschend herausgestellt, dass eine vergleichsweise kleine Winkelamplitude für die rotatorisch-oszillierende Bewegung des Reaktors ausreicht, um eine gute Durchmischung und/oder eine hinreichende Intensivierung von Transportprozessen zu erreichen. Insbesondere ist es kaum erforderlich, 3600°-Umdrehungen (das entspricht 10 Umdrehungen) des Reaktors zu realisieren, so dass konstruktiv aufwändige Lösungen für die Anbindung des oszillatorisch rotierenden Reaktors an die ruhende Umgebung (z.B. zur Zu- und Abfuhr von Medien und Gasen, von elektrischer Energie und von elektrischen Signalen) nicht erforderlich sind.

Bei der erfindungsgemäßen Verwendung wird der Reaktor mit einer Winkelamplitude $\alpha$ im Bereich von $2° \leq |\alpha| \leq 3600°$, bevorzugt $20° \leq |\alpha| \leq 180°$, besonders bevorzugt $45° \leq |\alpha| \leq 90°$ rotatorisch-oszillierend bewegt, wobei Abweichungen von $\pm 5°$ vorhanden sein können. Insbesondere gilt $|\alpha| = 60°$ als ganz besonders bevorzugt bei der Verwendung von besonders scherarmen oberflächenbegasten Bioreaktoren. In Summe überstreicht damit die oszillierende Bewegung einen Winkel von $2 |\alpha|$.

Versuche haben gezeigt, dass sich bei Erhöhung des Leistungseintrags Bewegungszustände in dem Reaktor einstellen können, bei denen Gasblasen in das Reaktormedium eingetragen werden. Für Zellen und/oder Mikroorganismen, die durch eine Blasenbegasung nicht geschädigt werden, kann auf diese Weise eine sehr einfache Gasversorgung realisiert werden.

Es hat sich gezeigt, dass eine unerwünschte Schaumentwicklung zunächst wie erwartet mit zunehmender Reaktorbewegung ansteigt, um dann aber nach Durchschreiten einer maximalen Schaumhöhe wieder auf gut beherrschbare Schaumhöhen von wenigen Zentimetern abzufallen. Die Ursache für dieses sehr erstaunliche Phänomen dieser Schaumzerstörens besteht darin, dass bei diesen Bewegungszuständen der Flüssigkeit nicht nur das im Kopfraum befindliche Gas, sondern auch der Schaum selbst von der Oberfläche eingesogen wird. Der Schaum wird durch das Wiedereinsaugen unter die Flüssigkeitsoberfläche ohne Aufbringung von Scherkräften schonend, d.h. unter strikter Vermeidung des Gasblasenzerplatzens, wieder aufgelöst. Insbesondere kann sich eine Wellenströmung einstellen, durch die ein Teil des an der Oberfläche befindlichen Reaktorinhalts in das Innere des Reaktiorinhalts gefördert wird. In diesem bevorzugten Reaktortyp kann somit eine Schaumbildung weitgehend unterdrückt werden und es kann gleichzeitig eine besonders schonende und effektive Oberflächenbegasung realisiert werden. Die Anwendung des oszillierenden Schaumzerstörers ist jedoch keineswegs auf oberflächenbegaste Reaktoren begrenzt, sondern lässt sich allgemein in blasenbegasten Reaktoren vorteilhaft zum Einsatz bringen.

Mit zunehmendem Reaktorvolumen wird es zunehmend schwieriger, über eine Oberflächenbegasung eine ausreichende Ver- und Entsorgung von Zellen und/oder Mikroorganismen mit/von gasförmigen Stoffen zu erreichen. In einer besonders bevorzugten Ausführungsform erfolgt daher eine Begasung des Reaktors über einen oder mehrere seitlich im Bodenbereich angebrachte Gasverteiler. Erfolgt der Einfachheit halber eine punktförmige Gaszufuhr über einen einzigen Gasverteiler, so steigen Gasblasen infolge der rotatorisch-oszillierenden Bewegung des Reaktors und der der äußeren Bewegung des Reaktors nachhinkenden Bewegung des Reaktormediums in Form von zickzackförmigen Linien auf. Durch die rotatorisch-oszillierende Bewegung wird also auch bei einer punktförmigen Einleitung eine Verteilung der Gasblasen entlang der Wand, in der der Gasverteiler angebracht ist, bewirkt. Gleichzeitig sorgt eine Zirkularströmung infolge des Dichteunterschieds zwischen dem Bereich des Reaktormediums, in dem Gasblasen aufsteigen, und dem von der Gasverteilerstelle entfernten Bereich für eine Umwälzung des Reaktorinhalts.

Die Mischwirkung der rotatorisch-oszillierenden Bewegung wird durch die eckige Form des Reaktors/Containers einerseits und den nach innen gewölbten Boden andererseits noch unterstützt.

Ein oder mehrere Gasverteiler werden bevorzugt über die Anschlussplatte im Bodenbereich in den Einwegreaktor eingebracht. Ein Gasverteiler kann für eine grobblasige Begasung als eine Öffnung mit einem Durchmesser von 0,5 mm bis 10 mm oder als kurzes perforiertes Schlauch- oder Rohrstück mit einem mittleren Porendurchmesser von 0,5 mm bis 1 mm ausgeführt sein. Bevorzugt wird eine feinblasige Begasung z.B. mittels einer Sinterkerze mit Sinter-korngrößen von 0,2 $\mu$m bis 50 $\mu$m verwendet. Begasungseinrichtungen können als kurze Rohrstücke rechtwinklig zu Behälterwand oder als längere Rohrstücke parallel zur Behälterwand ausgeführt sein, wobei letztere an einer Stelle mit Gas versorgt und je nach Länge an einer weiteren Stelle gehalten werden müssen.

Die Erfindung wird nachstehend anhand von Figuren näher erläutert, ohne sie jedoch auf die gezeigten Ausführungsformen zu beschränken.

Es zeigen:

Fig. 1(a), (b)   Perspektivische Darstellung des erfindungsgemäßen Containers

Fig. 2(a)   Schematische Darstellung des erfindungsgemäßen Containers im Querschnitt von oben

Fig. 2(b)   Schematische Darstellung des erfindungsgemäßen Containers im Querschnitt durch die in Fig. 2 (a) gezeigte Linie zwischen den Punkten A und B

Fig. 3(a), (b)   Perspektivische Darstellung des erfindungsgemäßen Containers mit einer Anschlussplatte

Fig. 4(a),(b)   Verschiedene Ausführungsformen einer Anschlussplatte

Fig. 5(a),(b)   Schematische Darstellung der Gasblasenverteilung in einem rotatorisch oszillierenden Einwegreaktor

Fig. 6   Perspektivische Darstellung einer begasten Kombination aus Container und Einwegreaktor

Fig. 7(a), (b)   Perspektivische Darstellung einer Kombination aus Container und Antriebseinheit

Fig. 8   Schematische Darstellung eines oberflächenbegasten Containers und Reaktors

Fig. 9(a), (b)   Schematische Darstellung erfindungsgemäßer Reaktoren

Fig. 10(a), (b), (c)   Schematische Darstellung eines Verfahrens zur Herstellung eines erfindungsgemäßen Reaktors

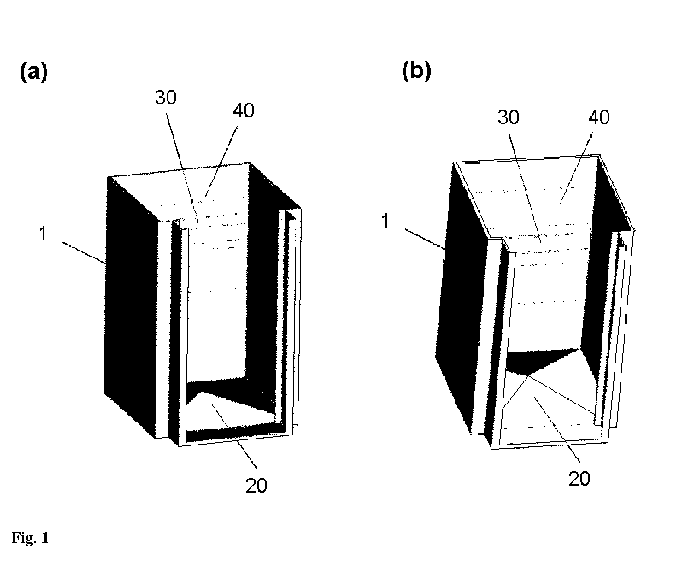

Figur 1 zeigt schematisch eine bevorzugte Ausführungsform des erfindungsgemäßen Containers 1 in einer perspektivische Darstellung von vorne (a) und schräg von oben (b). Der Container verfügt über einen pyramidal in den Innenraum 40 gewölbten Boden 20. Der Container weist einen Innenraum 40 zur Aufnahme eines Reaktors und einen Seitenkanal 30 zur Führung von Anschlussleitungen auf.

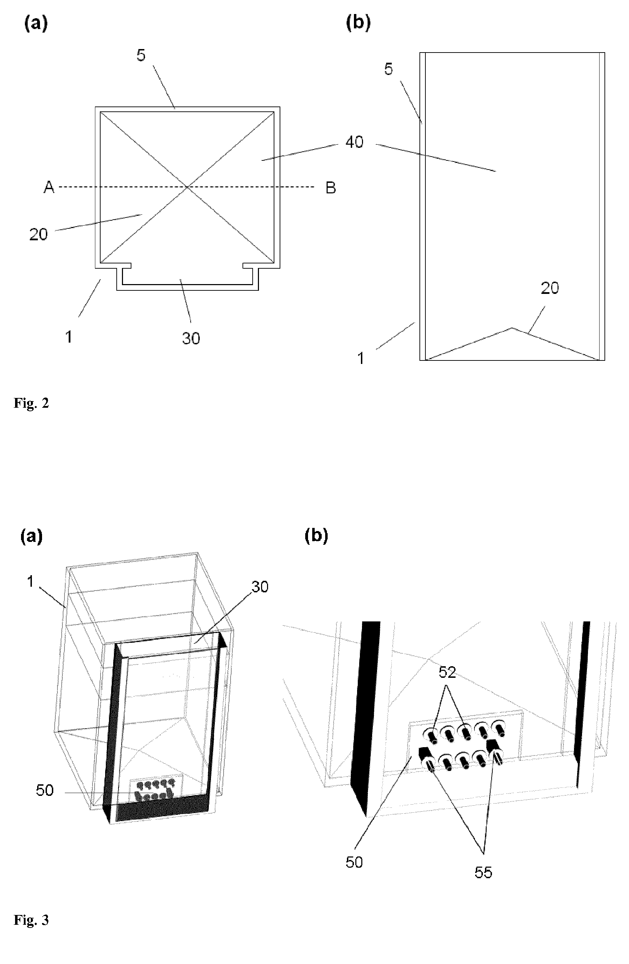

Figur 2(a) zeigt den Container 1 aus Figur 1 im Querschnitt von oben. Figur 2(b) zeigt den Container 1 im Querschnitt entlang der in Fig. 2(a) gezeigten gestrichelten Linie zwischen den Punkten A und B.

Figur 3(a) zeigt eine bevorzugte Ausführungsform des erfindungsgemäßen Containers 1 in einer perspektivischen Darstellung. Im Bodenbereich des Containers ist eine Anschlussplatte 50 angebracht. Der Bereich des Containers 1, der die Anschlussplatte 50 umfasst, ist in Figur 3(b) vergrößert dargestellt. Die Anschlussplatte 50 umfasst in der gezeigten Ausführungsform Anschlussstellen 52, die auf einer Seite mit einem Reaktor verbunden werden. Auf der anderen, dem Reaktor abgewandten Seite können z.B. Schlauchleitungen zur Medienversorgung angebracht werden. Ebenso kann die Anschlussplatte Anschlüsse für Sonden (z.B. pH-Sonde, Temperatursonde) aufweisen. In der gezeigten Ausführungsform sind Gasverteiler 55 in die Anschlussplatte integriert.

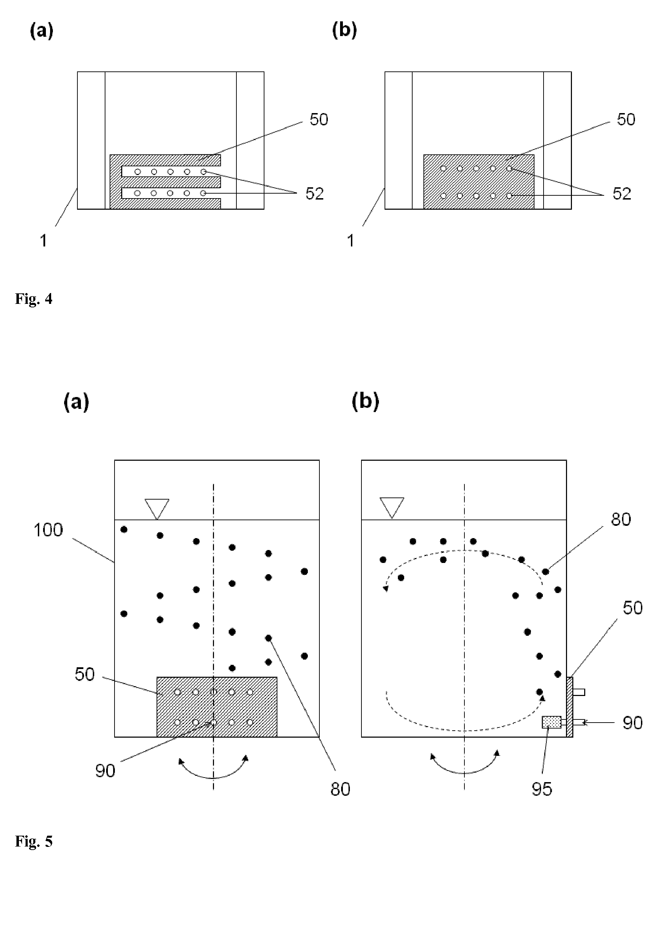

Figur 4 (a) und (b) zeigen verschiedene Ausführungsformen einer Anschlussplatte 50, die im Bodenbereich eines Container 1 angebracht sind. Die Anschlussplatte 50 in Figur 4(a) ist seitlich geöffnet und wird zur Fixierung des Reaktors im Container seitlich über die Anschlüsse 52 des Reaktors geschoben. Die Anschlussplatte kann wiederverwendet werden und ist Teil des Containers. Die Anschlussplatte 50 in Figur 4(b) verfügt über Durchführungen für das Hindurchführen der Anschlussleitungen. Sie wird bei der Montage mit dem Reaktor verbunden. In dieser Ausführungsform ist die Anschlussplatte selbst ebenfalls ein Einwegelement.

Figuren 5(a) und 5(b) zeigen in einer schematischen Darstellung die Begasung eines Reaktors über eine Anschlussplatte 50. In Figur 5(a) ist die Anschlussplatte auf den Betrachter gerichtet, in Figur 5(b) ist die Anschlussplatte 50 von der Seite gezeigt. Über einen Anschluss wird Gas 90 punktförmig in den Reaktor geführt. Hierfür wird vorzugsweise eine kurze Sinterkerze 95 verwendet, um eine feinblasige Begasung zu gewährleisten. Die rotatorisch-oszillierende Bewegung (angedeutet durch den Doppelpfeil) verhindert zur Erzeugung kleiner Gasblasen infolge des Anströmens der Begaser mit dem trägen Fluid die Blasenkoaleszenz an der Begaseroberfläche und bewirkt eine zickzackförmige Blasenaufgabe, d.h. die Gasblasen 80 steigen in Form einer Zickzack-Linie auf und werden somit über die gesamte Reaktorbreite verteilt. Die Begasung über die Reaktorwand führt zudem zu einer Zirkulationsströmung (dargestellt durch die gestrichelten Pfeile).

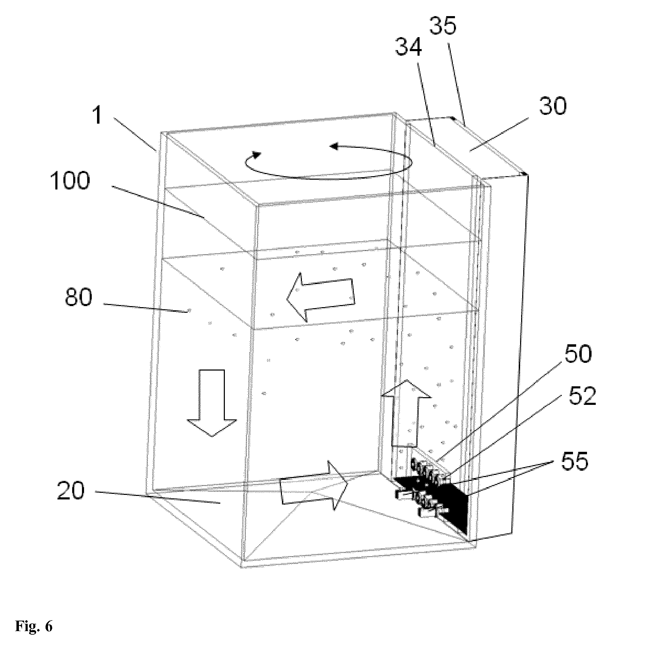

In Figur 6 ist die Begasung des Reaktors 100 in einem oszillatorisch bewegten Container 1 in einer perspektivischen Darstellung gezeigt. In dieser Ausführungsform wird über ein Gas zwei Gasverteiler 55 in den Reaktor gegeben. Die oszillatorisch rotierende Bewegung (dargestellt durch den Doppelpfeil) bewirkt eine Verteilung der Gasblasen 80. Die Zirkulationsströmung (dargestellt durch die dicken Pfeile), sorgt dafür, dass die Gasblasen auch in die Bereiche des Reaktors gelangen, die fern der Eintrittsstellen des Gases in den Reaktor liegen. Im Falle der Kultivierung von Zellen oder Mikroorganismen sorgt die Zirkulationsströmung dafür, dass die Zellen oder Mikroorganismen im Reaktor in der Schwebe gehalten werden. Durch den nach innen gewölbten Boden 20, der im vorliegenden Beispiel pyramidal ausgeführt ist, werden Wirbel erzeugt, die absinkende Zellen oder Mikroorganismen aufwirbeln und re-suspendieren.

Figur 6 zeigt auch eine bevorzugte Ausführungsform des Seitenkanals 30. Er verläuft zwischen den zwei beabstandeten, vorzugsweise transparent ausgeführten Wänden 34, 35 bis in den Bodenbereich des Reaktors. Die innere zum Rreaktor gerichtete Wand 34 dient der Stützung des vorzugsweise verformbaren Reaktors. Die äußere Wand 35 dient der Abdichtung des Containers nach außen. Beide Wände lassen sich zur frontalen Einführung des Reaktors in den Container entfernen.

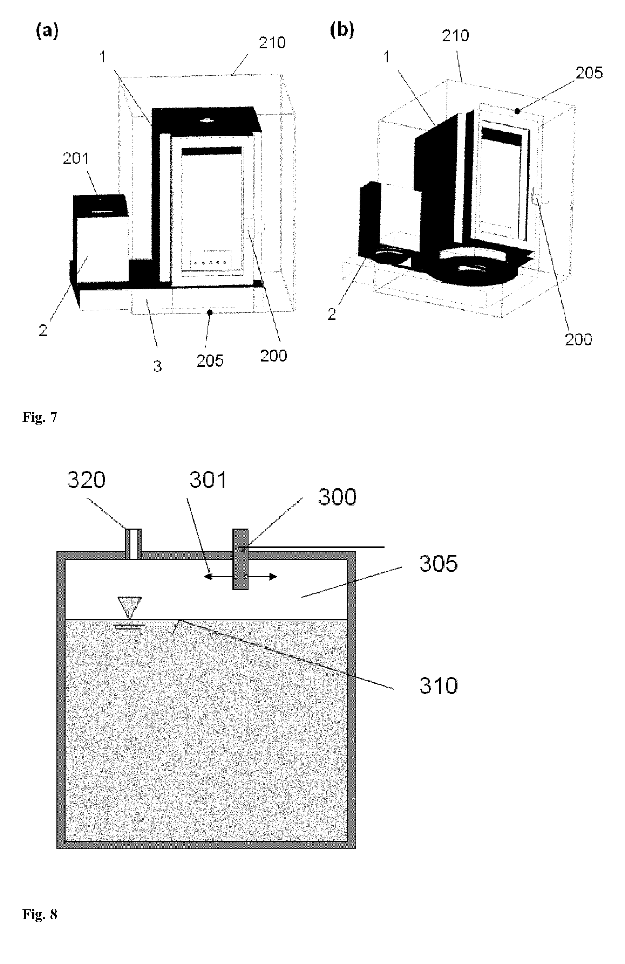

Figur 7 zeigt beispielhaft einen erfindungsgemäßen Container 1, der zusammen mit einer Antriebseinheit 2 auf einer gemeinsamen Bodenplatte 3 angebracht ist. Der Container 1 ist bezüglich einer vertikal stehenden Achse drehbar gelagert auf der Bodenplatte 3 angebracht. Die Antriebseinheit umfasst im vorliegenden Beispiel ein Pendelgetriebe, das über einen Zahnriemen mit dem Container verbunden ist und diesen in eine rotatorisch-oszillierende Bewegung versetzen kann.

In einer bevorzugten Ausführungsform ist der rotierende Container von einem Gestell 210 umgeben, das den Zugriff des Bedienungspersonal im laufenden Betrieb bei ordnungsgemäßer Verwendung sicher verhindert. Das Gestell 210 weist mindestens eine Tür 205 auf, die dem Bedienungspersonal nur bei gesichertem Betriebsstillstand den Zutritt zu Container und Reaktor gewährt. Türen 205 und Antriebseinheit sind durch ein geeignetes Schließsystem derart abgesichert, dass eine Inbetriebnahme des Containers nur bei geschlossenen Türen 205 ermöglicht wird. Bevorzugt wird dazu ein stromloses Sicherungssystem, dem Fachmann auch als Fortis-System bekannt, verwendet. Ein einziger verfügbarer, dedizierter Anlagenschlüssel schaltet bei Entfernen aus dem Schaltgehäuse 201 die Stromzufuhr der Antriebseinheit ab, bevor dieser für das Schließen der ausschließlich im geschlossenen Zustand der Tür 205 zu betätigenden Schließvorrichtung 200 verwendet werden kann. Der Vorteil der kodierten Einschlüsselverriegelung liegt in einer mechanischen Eigensicherheit ohne elektronische Überwachung. Eine Kabelverlegung durch die Hohlrohre des Gestells ist nicht notwendig. Dadurch ergeben sich Vorteile bei Reinigung, Abdichtung, Montage und Kosten.

Figur 8 zeigt beispielhaft die Gaszufuhr in einen oberflächenbegasten Reaktor. Um eine Kurzschlussströmung zwischen Gaszufuhr 300 und Gasabfuhr 320 zu verhindern, wird der Gasstrom mittels eines oder mehrerer Gasverteiler in horizontale Richtung umgelenkt und durch entsprechende Öffnungen unter Inkaufnahme von Druckverlusten auf höhere Geschwindigkeiten beschleunigt. Bevorzugte Druckverluste liegen zwischen 1 mbar und 5000 mbar. Besonders bevorzugt liegen die Druckverluste zwischen 10 mbar und 500 mbar. Mittels der auf diese Weise erzeugten horizontalen Gasstrahlen 301 wird eine einfache, kostengünstige und effiziente Vermischung des gesamten Gases im Kopfraum 305 des Reaktors ermöglicht.

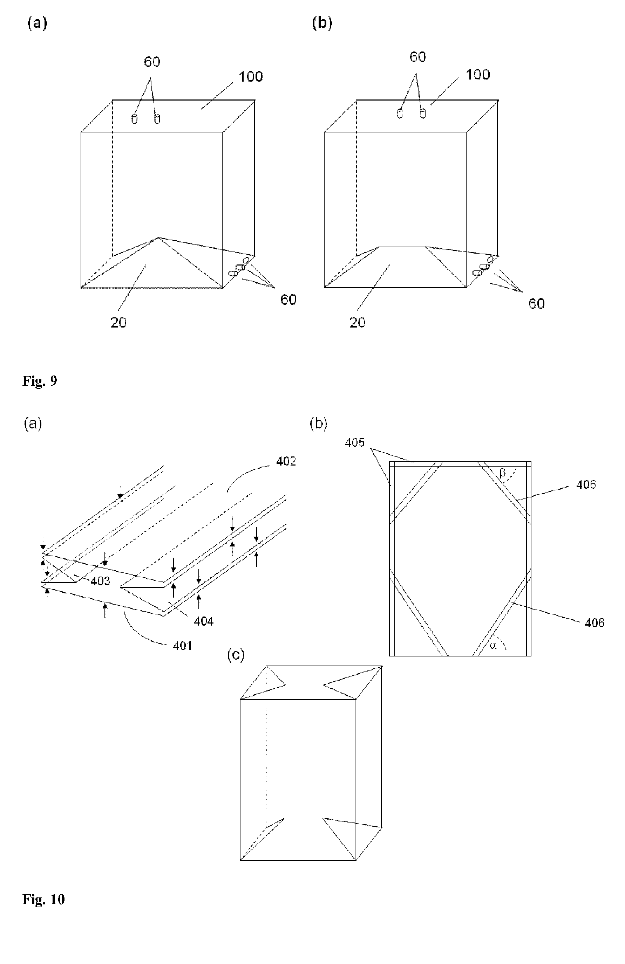

Figur 9 zeigt schematisch zwei polyederförmige Ausführungsbeispiele eines erfindungsgemäßen Reaktors 100. Der Reaktor umfasst einen Raum, der durch vorzugsweise flexibel ausgeführte (verformbare) Wände begrenzt wird. Der Boden 20 ragt in das Innere des Raumes hinein. Fig. 9 (a) zeigt einen pyramidal nach innen gestülpten/gewölbten Boden, der spitz zuläuft. In Fig. 9 (b) ist der obere Bereich der Pyramide als Kante ausgeführt.

In den Wänden sind Durchführungen und/oder Abschlüssen 60 angebracht, um eine Verbindung zwischen dem Innenraum des Reaktors und der Außenwelt zu schaffen.

Figur 10 zeigt schematisch, wie ein erfindungsgemäßer Reaktor aus Polymerfolien analog zu der in US6,186,932B1 beschriebenen Weise hergestellt werden kann. In dem Beispiel wird der Reaktor aus vier Folienteilen (401, 402, 403, 404) zusammengefügt. Dazu werden die Folien in der in Fig. 5(a) gezeigten Weise zusammengelegt: das Folienteil 401 liegt zuunterst, das Folienteil 402 liegt zuoberst die Folienteile 403 und 404 werden in gefalteter Form zwischen die Folienteile 401 und 402 gelegt. Die Folienteile werden an den Rändern ringsherum zusammengeschweißt (angedeutet durch die Pfeile), so dass ein geschlossener Beutel entsteht. Fig. 5(b) zeigt die Folienanordnung auf Fig. 5(a) von oben. Es sind Schweißnähte 405 rings um die Folienanordnung zu erkennen. Zusätzlich weist der Folienverbund Schweißnähte 406 auf, die über die Ecken verlaufen. Durch die Größen der Winkel α und β werden die Boden- und die Kopfform des Reaktors, im Fall des Bodens insbesondere die Größe der Bodenwölbung, bestimmt.

Die Ecken der Folienanordnung aus Fig. 5(b) werden nach Erzeugung der Eck-Schweißnähte 406 abgeschnitten.

Bezugszeichen:

| 1   | Container                  |
|-----|----------------------------|
| 2   | Antriebseinheit            |
| 3   | Bodenplatte                |
| 5   | Containerwand              |
| 20  | nach innen gewölbter Boden |
| 30  | Seitenkanal                |
| 34  | Kanalwand                  |
| 35  | Kanalwand                  |
| 40  | Innenraum                  |
| 50  | Anschlussplatte            |
| 52  | Anschlüsse                 |
| 55  | Gasverteiler               |
| 60  | Durchführungen / Anschlüsse |
| 80  | Gasblasen                  |
| 90  | Gaszufuhr                  |
| 100 | Reaktor                    |
| 200 | Schließvorrichtung         |
| 201 | Steuereinheit              |
| 205 | Tür                        |
| 210 | Gestell                    |
| 300 | Gaszufuhr                  |
| 301 | Gasstrahl                  |
| 305 | Kopfraum                   |
| 310 | Flüssigkeitsspiegel        |
| 320 | Gasabfuhr                  |
| 401 | Folienstück                |
| 402 | Folienstück                |
| 403 | Folienstück                |
| 404 | Folienstück                |

405     Schweißnaht längsseitig

406     Schweißnaht über Eck

**Patentansprüche**

1. Reaktor umfassend Wände, die einen Innenraum einschließen, und Durchführungen und/oder Anschlüsse in den Wänden, die eine Verbindung des Innenraums mit der äußeren Umgebung gestatten, wobei der Reaktor in mit Flüssigkeit gefülltem Zustand einen eckigen Querschnitt parallel zur Flüssigkeitsoberfläche und einen in den Innenraum gewölbten Boden aufweist und um eine innerhalb des Reaktors ortsfeste, vertikale Achse rotatorisch-oszillierend in Bewegung versetzbar ist, **dadurch gekennzeichnet, dass** der Reaktor ein oder mehrere Gasverteiler im Seitenbereich des Reaktors im Bodenbereich nahe der Bodenkante in den Reaktor aufweist.

2. Reaktor nach Anspruch 1, **dadurch gekennzeichnet, dass** der nach innen gewölbte oder wölbbare Boden über Kanten verfügt, die in den Innenraum des Reaktors hineinragen.

3. Reaktor nach Anspruch 1 oder 2, **dadurch gekennzeichnet, dass** der nach innen gewölbte oder wölbbare Boden pyramidal ausgeführt ist.

4. Reaktor nach einem der Ansprüche 1 bis 3, **dadurch gekennzeichnet, dass** die Höhe $h$ der Bodenwölbung im Verhältnis zum kreisäquivalenten Durchmesser d des Bodens im Bereich von 3% bis 100 % liegt.

5. Reaktor nach einem der Ansprüche 1 bis 4, **dadurch gekennzeichnet, dass** der Gasverteiler als eine Öffnung mit einem Durchmesser von 0,5 mm bis 10 mm, als kurzes perforiertes Schlauch- oder Rohrstück mit einem mittleren Porendurchmesser von 0,5 mm bis 1 mm oder als eine Sinterkerze mit Sinterkorngrößen von 0,2 $\mu$m bis 50 $\mu$m ausgeführt ist.

6. Reaktor nach einem der Ansprüche 1 bis 5, **dadurch gekennzeichnet, dass** der Gasverteiler als kurze Rohrstücke rechtwinklig zu Behälterwand oder als längere Rohrstücke parallel zur Behälterwand ausgeführt ist, wobei letztere an einer Stelle mit Gas versorgt und je nach Länge an einer weiteren Stelle gehaltert ist.

7. Reaktor nach einem der Ansprüche 1 bis 5, **dadurch gekennzeichnet, dass** die Wände flexibel und der Reaktor somit verformbar ausgeführt ist.

8. Vorrichtung mindestens umfassend einen Reaktor nach einem der Ansprüche 1 bis 7, eine Antriebseinheit zur Erzeugung einer rotatorisch-oszillierenden Bewegung des Reaktors um eine innerhalb des Reaktors ortsfeste, vertikale Achse und einen Container zur Aufnahme des Reaktors, falls der Reaktor verformbar ausgeführt ist.

9. Verwendung einer Vorrichtung nach einem Anspruch 8 zur Kultivierung von Zellen und/oder Mikroorganismen.

10. Verfahren zur Kultivierung von Zellen und/oder Mikroorganismen in einem Reaktor nach einem der Ansprüche 1 bis 7 oder in einer Vorrichtung nach Anspruch 8, wobei der Reaktor mit Flüssigkeit unter Wahrung seiner hydrodynamischen und prozesstechnischen Eigenschaften gefüllt wird und der Reaktor um eine im Reaktor ortsfeste, vertikale Achse rotatorisch-oszillierend in Bewegung versetzt wird und mittels der Gasverteiler im Seitenbereich des Reaktors im Bodenbereich nahe der Bodenkante in den Reaktor begast wird.

11. Verfahren nach Anspruch 10, **dadurch gekennzeichnet dass** im Reaktor ein Verhältnis von Flüssigkeitsspiegel zur Reaktorbreite von 0,2 bis 2.

12. Verfahren nach einem der Ansprüche 10 oder 11, **dadurch gekennzeichnet, dass** der Reaktor mit einen Kopfraum zwischen Reaktorkopf und Flüssigkeitsspiegel von mindestens 5% bis 50 % Flüssigkeitshöhe betrieben wird.

13. Verfahren nach einem der Ansprüche 10 bis 12, **dadurch gekennzeichnet, dass** der Reaktor mit einer Winkelamplitude $\alpha$ im Bereich $45° \leq |\alpha| \leq 90°$ rotatorisch-oszillierend bewegt wird und die oszillierende Bewegung einen Winkel von $2\,|\alpha|$ überstreicht, wobei Abweichungen von $\pm 5°$ vorhanden sein können.

14. Verfahren nach einem der Ansprüche 10 bis 13, **dadurch gekennzeichnet, dass** Beschleunigen und Abbremsen der Reaktorrotation mit einer im Wesentlichen konstanten Winkelbeschleunigung bzw. -verzögerung erfolgt.

**(a)**

30

40

1

20

**(b)**

30

40

1

20

Fig. 1

**(a)**

5

40

A --------- B

20

1        30

**(b)**

5

40

20

1

Fig. 2

**(a)**

1

30

50

**(b)**

52

50

55

Fig. 3

**(a)**

**(b)**

50

52

1

50

52

1

**Fig. 4**

**(a)**

**(b)**

100

50

90

80

80

50

90

95

**Fig. 5**

**Fig. 6**

**(a)**

**(b)**

Fig. 7

Fig. 8

(a)

(b)

Fig. 9

(a)

(b)

(c)

Fig. 10

**EP 2 517 786 A2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007121958 A1
- US 6186932 B1